# EUROPÄISCHE PATENTSCHRIFT

(11) **EP 1 467 980 B1**
(45) Veröffentlichungstag und Bekanntmachung des Hinweises auf die Patenterteilung: **27.07.2005**
(21) Anmeldenummer: 03708046.2
(22) Anmeldetag: 03.01.2003
(51) Int. Cl.: C07D 277/36, C07C 21/18, A01N 43/78

(54) **VERFAHREN ZUR HERSTELLUNG VON HALOGENIERTEN 2-(3-BUTENYLSULFANYL)-1, 3- THIAZOLEN**
METHOD FOR PRODUCING HALOGENATED 2-(3-BUTENYLSULFANYL)-1,3-THIAZOLES
PROCEDE DE PRODUCTION DE 2-(3-BUTENYLSULFANYL)-1,3-THIAZOLES HALOGENES

(30) Priorität: 15.01.2002 DE 10201238
(43) Veröffentlichungstag der Anmeldung: 20.10.2004
(73) Patentinhaber: Bayer CropScience AG, 40789 Monheim (DE)
(72) Erfinder: STRAUB, Alexander, 42117 Wuppertal (DE)
(86) Internationale Anmeldenummer: PCT/EP2003/000028
(87) Internationale Veröffentlichungsnummer: WO 2003/059896

(56) Entgegenhaltungen:
- EP-A- 0 926 140
- WO-A-88/00183
- US-A- 3 510 503
- PATENT ABSTRACTS OF JAPAN & JP 59 155355 A (SHIONOGI & CO LTD), 4. September 1984 (1984-09-04)
- C.L. JENKINS, J.K. KOCHI: JOURNAL OF ORGANIC CHEMISTRY, Bd. 36, Nr. 21, 1971, Seiten 3103-3111, XP002243668
- M.T. BOGERT: JOURNAL OF THE AMERICAN CHEMICAL SOCIETY, Bd. 25, 1903, Seiten 289-291, XP002243669

## Beschreibung

Die vorliegende Erfindung bezieht sich auf ein Verfahren zur Herstellung von halogenierten 2-(3-Butenylsulfanyl)-1,3-thiazolen der Formel (I), 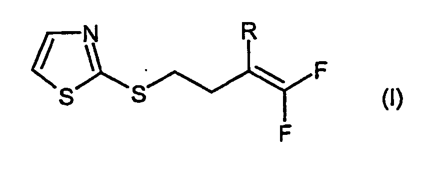 worin
- R: für H oder F steht

2-[(3-Butenyl)sulfanyl]-1,3-thiazole der Formel (I) sind wichtige Vorstufen für die Herstellung von Schädlingbekämpfungsmitteln, wie sie z.B. in WO 01/02378, US 3,513,172, US 3,697,538 oder WO 95/24403 beschrieben werden. Die Verbindung 2-[(3,4,4-Trifluor-3-butenyl)sulfanyl]-1,3-thiazol wurde in WO 86/07590 beschrieben. Die Verbindung 2-[(4,4-Difluor-3-butenyl)sulfanyl]-1,3-thiazol wurde z.B. in WO 95/24403 beschrieben.

Die Herstellung von 2-[(3,4,4-Trifluor-3-butenyl)sulfanyl]-1,3-thiazol oder 2-[(4,4-Difluor-3-butenyl)sulfanyl]-1,3-thiazol erfolgte bislang gemäß WO 86/07590 durch Metallierung von Thiazol mit n-Butyllithium, Umsetzung mit elementarem Schwefel und anschließender Umsetzung mit 4-Brom-1,1,2-trifluor-1-buten (siehe Bsp. 16 aus WO 86/07590) oder durch Alkylierung von 2-Mercaptothiazol mit 4-Brom-1,1,2-trifluor-1-buten (siehe auch WO 01/02378). Ein entsprechender Ansatz wird auch bei der Herstellung von 2-[(4,4-Difluor-3-butenyl)sulfanyl]-1,3-thiazol verfolgt (siehe WO 98/47884 oder WO 95/04727).

Für die Herstellung der hierzu benötigten Vorstufe 2-Mercapto-1,3-thiazol sind bereits eine Reihe von Verfahren beschrieben worden (WO 98/37074, EP 0 926 140 Al, US 5,994,553, US 2,426,397, Mathes et al. (1948), *J. Am. Chem. Soc.* 70, 1451). In den hier beschriebenen Verfahren wird jeweils die Umsetzung eines Salzes der Dithiocarbaminsäure, insbesondere das Ammoniumdithiocarbamat mit einer wässrigen Lösung von Chloracetaldehyd unter sauren oder neutralen Bedingungen beschrieben.

Diese Dithiocarbamate und deren Säuren sind jedoch instabil (Gattow et al. (1969) *Z. Anorg. Allg. Chem*. 365, 70; Gattow und Hahnlcamm (1969), *Z. Anorg. Allg. Chem*. 364, 161; Redemann et al. (1955) *Org. Synth. Coll. Vol*. 3, 763; Fourquet (1969) *Bull. Soc. Chim. Fr.* 3, 301; Hahnkamm et al. (1969) *Z. Anorg. Allg. Chem.* 368, 127). Das hat zur Folge, dass es zu erheblichen Nebenreaktionen und zur Freisetzung von gefährlichem Schwefelkohlenstoff kommen kann. Die Ausbeuten sind entsprechend schlecht. Hinzu kommt, dass die Synthese von Ammoniumdithiocarbamat mit dem Einsatz des gefährlichen Schwefelkohlenstoffs und des gasförmigen Ammoniaks verbunden ist.

Aufgabe der vorliegenden Erfindung war es deshalb, ein Verfahren zu entwickeln, welches eine Alternative zu den genannten Verfahren darstellt und unter Umgehung der Zwischenstufe 2-Mercapto-1,3-thiazol direkt zu den gewünschten Endprodukten der Formel (I) führt.

Es war bekannt, dass sich Arylthiocyanate mittels Schwefelwasserstoff in S-Aryldithiocarbamate überführen lassen und diese mit Chloroacetaldehyddiethylacetal zu 2-Arylmercaptothiazolen cyclisiert werden können (Maeda et al. (1983), *Chem. Pharm. Bull.* 31, 3424, siehe Chart 3, VII). Die Cyclisierung von Methyldithiocarbamat mit Chloracetaldehyd ist ebenfalls eine bekannte Reaktion (Brandsma et al.(1985) *Synthesis*, 948). Die nachstehend genannte Verbindung der Formel V, in welcher R für F steht, ist aus US 3,510,503 bekannt.

Es wurde nun gefunden, dass man Verbindungen der Formel (I) herstellen kann, indem man
(a) eine Verbindung der Formel (II) 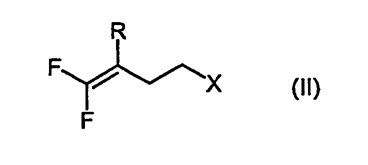 worin
   - R: für H oder F und bevorzugt für F steht, und
   - X: für Brom, Chlor, Mesylat oder Tosylat und bevorzugt für Brom steht,
   mit einem Rhodanidsalz der Formel (III)

   M⁺SCN⁻ (III)

   worin
   - M⁺: für Wasserstoff, für ein Ammoniumion, ein Tetraalkylammoniumion oder ein Alkalimetallion, z.B. K⁺ oder Na⁺ steht,
   zu 3-Butenyl-thiocyanaten der Formel (IV) 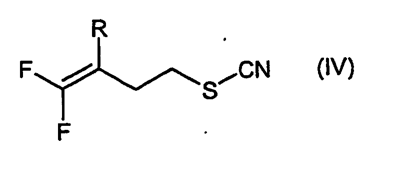 worin
   - R: die vorstehend angegebene Bedeutung hat,
   gegebenenfalls in Gegenwart eines Reaktionshilfsmittels und gegebenenfalls in Gegenwart eines Verdünnungsmittels
   umsetzt,
(b) diese anschließend durch Zuführung von Schwefelwasserstoff oder von Salzen des Schwefelwasserstoffs,
   gegebenenfalls in Gegenwart eines Reaktionshilfsmittels und gegebenenfalls in Gegenwart eines Verdünnungsmittels,
   in das 3-Butenyl-1-dithiocarbamat der Formel (V) 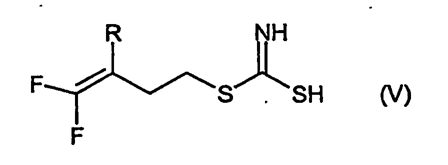 worin
   - R: die vorstehend angegebene Bedeutung hat,
   überführt, und
(c) dieses schließlich, gegebenenfalls in Gegenwart eines Reaktionshilfsmittels und gegebenenfalls in Gegenwart eines Verdünnungsmittels, mit Acetaldehyd, Chloracetaldehyd (ClCH₂CHO) oder dessen Acetale, z.B. Chloracetaldehyddialkylacetal, umsetzt. Bei den Acetalen kann es sich auch um cyclische Acetale handeln.
Das erfindungsgemäße Verfahren hat den Vorteil, dass auf die Verwendung des instabilen Ammoniumdithiocarbamats verzichtet werden kann. Die Zwischenstufen der Formel (IV) und (V) sind dagegen lagerstabil. 3,4,4-Trifluor-3-butenyl-1-dithiocarbamat der Formel (V) lässt sich z.B. entweder mit Acetaldehyd, Chloracetaldehyd oder dessen Acetalen sehr leicht zum 2-[(3,4,4-Trifluor-3-butenyl)sulfanyl]-1,3-thiazol umsetzen, ohne dass man Zersetzung und Freisetzung von gefährlichem CS₂ befürchten muss und ohne dass harzige Nebenprodukte auftreten, wie sie bei der Umsetzung von Ammoniumdithiocarbamat mit Chloracetaldehyd auftreten. Solche Nebenprodukte werden auch in EP 0 926 140 Al erwähnt. Auf das in den bisherigen Verfahren verwendete oxidationsempfindliche 2-Mercapto-1,3-thiazol kann verzichtet werden. Bei der Umsetzung von Verbindungen der Formel (V) mit (cyclischen) Acetalen ist die Gegenwart einer Säure, wie z.B. HCl, p-Toluolsulfonsäure oder Methansulfonsäure von Bedeutung.

Ein weitere Besonderheit des Verfahrens ist in der Umsetzung von 3-Butenyl-thiocyanaten der Formel (IV) mit Schwefelwasserstoff bzw. mit dessen Salzen zu sehen. Schwefelnucleophile; wie z.B. das H₂S addieren in der Regel an die Doppelbindung fluorhaltiger Olefine (z.B. Ali et al. (1982), J. Med. Chem. 25, 1235). In der vorliegenden Erfindung konnte gezeigt werden, dass die Addition von H₂S bzw. von dessen Salzen (Reaktion (b)) an Verbindungen der Formel (IV) überraschenderweise ausschliesslich an die Thiocyanatgruppe und nicht an das Olefin erfolgt. Diese hier beschriebene Reaktion ist ebenfalls Gegenstand der Erfindung.

Aus dem gleichen Grund ist es auch als überraschend anzusehen, dass bei der Reaktion eines Rhodanidsalzes der Formel (III) (M⁺SCN⁻) und einer Verbindung der Formel (II) nur die Substitution des Restes X, nicht aber die Addition an die Doppelbindung erfolgt. Für eine Reaktion von Verbindungen der Formel (II) mit Salzen der Rhodansäure der Formel (III) war es ferner nicht zu erwarten, dass das gewünschte Thiocyanat der Formel (IV) auf diese Weise erhalten werden kann.

Bekannt ist bislang vielmehr, dass bei Umsetzungen von Alkylhalogeniden mit Rhodansäuresalzen häufig die thermodynamisch stabileren Isothiocyanate entstehen (vgl. Houben-Weyl Bd. IX, S. 857 und S. 867). Es ist daher als außergewöhnlich zu bezeichnen, dass im vorliegenden Fall ausschließlich das gewünschte Regioisomer 3-Butenyl-thiocyanat erhalten wird. Die beschriebene Reaktion (a) ist ebenfalls Gegenstand der vorliegenden Erfindung.

Alternativ zum oben genannten Verfahren ist es auch möglich, Verbindungen der Formel (II) direkt mit Ammoniumdithiocarbamat (H₂NCS₂NH₄) zu 3-Butenyl-1-dithiocarbamaten der Formel (V) umzusetzen.

Das erfindungsgemäße Verfahren sowie der vorstehend genannte alternative Schritt lassen sich schematisch wie folgt darstellen:

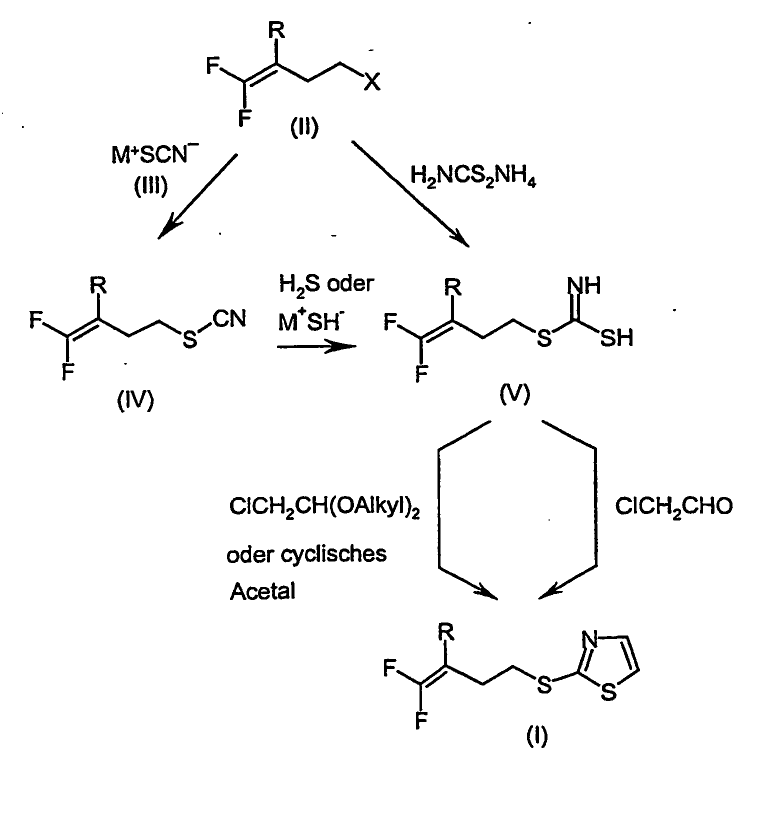

Verwendet man beispielsweise 4-Brom-1,1,2-trifluor-1-buten und Ammoniumrhodanid erhält man 3,4,4-Trifluor-3-butenylthiocyanat, das dann mit H₂S zu 3,4,4-Trifluor-3-butenyl-dithiocarbamat umgesetzt wird. Durch die weitere Umsetzung des 3,4,4-Trifluor-3-butenyl-dithiocarbamats mit Chloracetaldehyd erhält man dann 2-[(3,4,4-Trifluor-3-butenyl)sulfanyl]-1,3-thiazol. Dieser Reaktionsablauf kann schematisch wie folgt dargestellt werden:

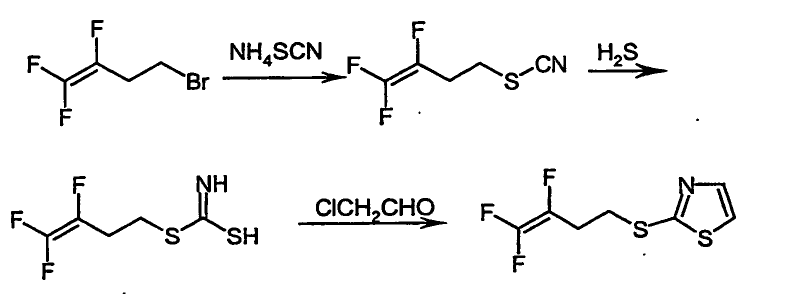

Die erfindungsgemäßen Verfahrensschritte können jeweils direkt aufeinander folgen oder auch jeweils einzeln durchgeführt werden, wobei das jeweilige Produkt auch gereinigt werden kann.

Die beim erfindungsgemäßen Verfahren (a) verwendeten Verbindungen zur Herstellung der Verbindungen der Formel (IV) sind durch die Formel (II) charakterisiert. In der Formel (II) steht X insbesondere bevorzugt für Brom und R insbesondere bevorzugt für Fluor. Die Verbindungen der Formel (II) sind bekannte Verbindungen. Verbindungen der Formel (II) in welcher X für F steht, werden z.B. in WO 86/07590 beschrieben und können nach dem dort angegebenen Verfahren hergestellt werden. Verbindungen der Formel (II) in welcher X für H steht, werden z.B. in GB 2304713 beschrieben und können nach dem dort angegebenen Verfahren hergestellt werden.

Die beim erfindungsgemäßen Verfahren (a) weiter verwendeten Verbindungen zur Herstellung der Verbindungen der Formel (IV) sind durch die Formel (III) charakterisiert. In der Formel (III) steht M⁺ bevorzugt für Kalium-, Natrium- oder Ammoniumionen, insbesondere bevorzugt für Ammoniumionen. Die Rhodanidsalze (Thiocyanate) der Formel (III) wie z.B. das Ammoniumrhodanid (NH₄SCN) sind bekannte Verbindungen und sind kommerziell erhältlich.

Die Verbindungen der Formel (IV), die auch Ausgangsstoffe für die Herstellung der Verbindungen der Formel (V) sind, sind als neue Substanzen auch Gegenstand der vorliegenden Anmeldung. In der Formel (IV) steht R bevorzugt für Wasserstoff oder Fluor, besonders bevorzugt für Fluor.

Die Verbindungen der Formel (IV) können nach dem vorstehend genannten Verfahren (a) hergestellt werden. Die Herstellung von Trifluorbutenyldithiocarbamaten wurde z.B. in US 3,510,503 beschrieben, wobei dort die Umsetzung eines 4-Brom-1,1,2-trifluor-1-butens unter anderem mit einem substituierten Ammoniumdithiocarbamat erfolgt oder wobei nach Kondensation eines geeigneten Mercaptans und CS₂ unter basischer Katalyse die Zugabe von 4-Brom-1,1,2-trifluor-1-buten erfolgt.

Die beim erfindungsgemäßen Verfahren (b) weiter verwendeten Verbindungen zur Herstellung der Verbindungen der Formel (V) sind Schwefelwasserstoff (H₂S) sowie dessen Salze. Diese Verbindungen können allgemein mit den Formeln MSH und M₂S beschrieben werden, worin M⁺ z.B. für Ammoniumionen oder Natriumionen steht. Bevorzugt werden im erfindungsgemäßen Verfahren (b) H₂S, Ammoniumsulfid, NaSH-Lösung oder auch Na₂S eingesetzt.

Das beim alternativen erfindungsgemäßen Verfahren verwendbare Ammoniumsalz der Dithiocarbaminsäure (NH₂NCS₂NH₄) zur Herstellung der Verbindung (V) ausgehend von Verbindungen der Formel (II) ist bekannt und auch kommerziell erhältlich.

Das beim erfindungsgemäßen Verfahren (c) zur Herstellung von Verbindungen der Formel (I) verwendete Chloracetaldehyd bzw. Chloracetaldehyddialkylacetale sind bekannt und kommerziell erhältlich.

Die beim erfindungsgemäßen Verfahren (c) weiter verwendeten Verbindungen zur Herstellung der Verbindungen der Formel (I) sind durch die Formel (V) charakterisiert. Die Verbindungen der Formel (V) sind als neue Verbindungen Gegenstand der vorliegenden Anmeldung. Sie können nach den vorstehend genannten Verfahren (b) bzw. (a) und (b) hergestellt werden. Die Verbindungen der Formel (V) können als Tautomere vorliegen (siehe Darstellung oben und in Beispiel 2).

Die erfindungsgemäßen Verfahren (a) bis (c) zur Herstellung der Verbindungen der allgemeinen Formel (I) werden vorzugsweise unter Verwendung von Verdünnungsmitteln durchgeführt. Als Verdünnungsmittel zur Durchführung des erfindungsgemäßen Verfahrens kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran, 2-Methyl-tetrahydrofuran, Methyl-t-butylether oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether oder deren Gemische mit Wasser.

Als Verdünnungsmittel zur Durchführung des Verfahrens (a) kommen insbesondere protisch-polare Lösungsmittel wie z.B. Alkohole, wie Methanol, Ethanol, n- oder i-Propanol, Ethylenglykolmonomethylether, Ethylenglykolmonoethylether, Diethylenglykolmonomethylether oder Diethylenglykolmonoethylether in Frage. In einer besonders bevorzugten Ausführungsform wird als Verdünnungsmittel Ethanol verwendet.

Als Verdünnungsmittel zur Durchführung des Verfahrens (b) kommen insbesondere z.B. Ether, wie Diethylether, Düsopropylether, Methyl-t-butylether, Dioxan, Tetrahydrofuran, 2-Methyl-tetrahydrofuran, Ethylenglykoldimethyl- oder -diethylether, Pyridin, Essigsäureethylester oder Isopropylacetat in Frage. In einer besonders bevorzugten Ausführungsform wird als Verdünnungsmittel Methyl-*tert*-butylether eingesetzt.

Als Verdünnungsmittel zur Durchführung des Verfahrens (c) kommen insbesondere z.B. Dioxan, Acetonitril und Carbonsäuren, wie z.B. Eisessig, p-Toluolsulfonsäure, Methansulfonsäure oder Gemische davon in Frage. Die Carbonsäuren, p-Toluolsulfonsäure oder Methansulfonsäure werden bevorzugt auch nur in katalytischen Mengen zugesetzt.

Als Reaktionshilfsmittel für die erfindungsgemäßen Verfahren (a) und (b) kommen die im Allgemeinen üblichen anorganischen oder organischen Basen oder Säureakzeptoren in Betracht. Hierzu gehören vorzugsweise Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische Stickstoffverbindungen, wie beispielsweise Ammoniak, Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, 1,4-Diazabicyclo[2.2.2]-octan (DABCO), 1,5-Diazabicyclo[4.3.0]-non-5-en (DBN), oder 1,8-Diazabicyclo[5.4.0]-undec-7-en (DBU).

Für das Verfahren (b) werden bevorzugt 0,1-20 mol-% Base verwendet, besonders bevorzugt 0,5-8 mol-% Base.

Als Reaktionshilfsmittel für das erfindungsgemäße Verfahren (c) kommen insbesondere z.B. Carbonsäuren, wie z.B. Eisessig, HCl, BF₃, H₂SO₄, Trifluoressigsäure, p-Toluolsulfonsäure oder Methansulfonsäure in Betracht, die direkt als Lösungsmittel verwendet werden, der Reaktion jedoch auch in katalytischen Mengen zugesetzt werden können. Bevorzugt liegen dabei 0,1 bis 10 mol-%, besonders bevorzugt 0,5 bis 5 mol-% der genannten Reaktionshilfsmittel vor.

Die Reaktionstemperaturen können bei der Durchführung des erfindungsgemäßen Verfahrens in einem größeren Bereich variiert werden. Im Allgemeinen arbeitet man bei Temperaturen zwischen 0°C und 150°C, vorzugsweise zwischen 10°C und 120°C. Bevorzugte Temperaturbereiche können auch den Herstellungsbeispielen entnommen werden.

Die erfindungsgemäßen Verfahren werden im Allgemeinen unter Normaldruck durchgeführt. Es ist jedoch auch möglich, die erfindungsgemäßen Verfahren unter erhöhtem oder vermindertem Druck - im Allgemeinen zwischen 0,1 bar und 50 bar, bevorzugt zwischen 1 und 10 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Bei der Durchführung des erfindungsgemäßen Verfahrens (b) werden vorzugsweise pro Mol der Verbindung der Formel (IV) 1,5 bis 3 Mol Schwefelwasserstoff bzw. ein Salz desselben verwendet. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

In einer bevorzugten Ausführungsform des erfindungsgemäßen Verfahrens (a) zur Herstellung der Verbindungen der Formel (IV) wird das Rhodanid der Formel (III) in Lösungsmittel, bevorzugt Alkohol, mit einer Verbindung der Formel (II) gerührt, bevorzugt bei Raumtemperatur, wobei ein Niederschlag entsteht. Anschließend wird der Ansatz unter Rückfluss erhitzt und dann abgekühlt, das Filtrat abgesaugt und eingedampft.

In einer bevorzugten Ausführungsform des erfindungsgemäßen Verfahrens (b) zur Herstellung der Verbindungen der Formel (V) wird in eine Lösung der Verbindung der Formel (IV), bevorzugt in Methyl-t-butylether, bei Anwesenheit einer Base, bevorzugt Triethylamin, langsam Schwefelwasserstoff bis zur Sättigung eingeleitet und anschließend gerührt. Nach Verdampfen des Lösungsmittels wird die Verbindung der Formel (V) erhalten.

In einer bevorzugten Ausführungsform des erfindungsgemäßen Verfahrens (c) zur Herstellung der Verbindungen der Formel (I) wird die Verbindung der Formel (V) in Verdünnungsmittel, bevorzugt Dioxan, mit einer wässrigen Chloracetaldehydlösung bei Anwesenheit einer katalytischen Menge Salzsäure versetzt und unter Schutzgas gekocht. In einer weiteren bevorzugten Ausführungsform des erfindungsgemäßen Verfahrens (c) zur Herstellung der Verbindungen der Formel (I) wird die Verbindung der Formel (V) in Verdünnungsmittel, bevorzugt Eisessig, mit Chloracetaldehyddiethylacetal und bei Anwesenheit einer katalytischen Menge von p-Toluolsulfonsäuremonohydrat versetzt und bei erhöhter Temperatur gerührt. Nach Abkühlung auf Raumtemperatur wird eingedampft, der Rückstand z.B. in Dichlormethan aufgenommen und mit Lauge, bevorzugt 1 N Natronlauge gewaschen und die organische Phase abgetrennt.

Die Herstellung der Verbindungen der Formel (I) geht auch aus den nachfolgenden Beispielen hervor, die die Verfahrensschritte (a), (b) und (c) weiter illustrieren. Die Beispiele sind jedoch nicht in einschränkender Weise zu interpretieren.

### Beispiele

### Beispiel 1: Herstellung von 3,4,4-Trifluoro-3-butenylthiocyanat (Verfahren (a))

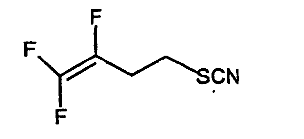

60,25 g (771,7 mmol) Ammoniumrhodanid wird in 400 ml Ethanol mit 164,3 g (810,3 mmol) 4-Brom-1,1,2-trifluor-1-buten versetzt und 2h bei Raumtemperatur gerührt, wobei ein weißer Niederschlag entsteht. Anschließend wird 8 h zum Rückfluss gekocht. Nach Abkühlen auf Raumtemperatur wird abgesaugt und das Filtrat im Vakuum eingedampft. Der Eindampfrückstand wird in Dichlormethan aufgenommen, mit Wasser gewaschen, mit Natriumsulfat getrocknet und im Vakuum eingedampft. Man erhält 121,1 g (93,3 % der Theorie; Gehalt nach GC/MS: 99,4 Flächen-%) eines gelben Öls.
R_{f} (SiO₂, CH₂Cl₂): 0,85.
¹H-NMR (CDCl₃, 400 MHz):δ=2,8-2,92 (m, 2H), 3,1 (t, 1H).
¹³C-NMR (CDCl₃, 100 MHz): δ=26,6 (CH₂-CF), 29,5 (S-CH₂), 110,9 (SCN), 125,4
(CF), 153,7 (CF₂).
MS (DCI,NH₃): 167 (11 %, M⁺), 108 (92%), 95 (100%), 69 (40%).

### Beispiel 2: Herstellung von 3,4,4-Trifluoro-3-butenyldithiocarbamat (Rhodanidverfahren) (Verfahren (b))

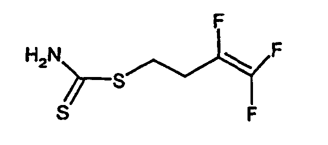

Man leitet in eine Lösung von 33,41 g (200 mmol) 3,4,4-Trifluoro-3-butenylthiocyanat und insgesamt 0,26 g Triethylamin in 130 ml Methyl-*tert*.-butylether bei 10°C langsam Schwefelwasserstoff bis zur Sättigung ein (ca. 2 Moläquivalente) und rührt anschließend bei Raumtemperatur über Nacht Nach Verdampfen des Lösungsmittels im Vakuum erhält man 41,2 g (88,5 % der Theorie; Gehalt nach GC/MS: 86,4 Flächen-%) eines kristallisierenden Rückstands, der aus kaltem Toluol umkristallisiert werden kann.
Smp.: 49-53°C
R_{f} (SiO₂, Toluol-Essigester 4:1): 0,5.
¹H-NMR (CDCl₃, 400 MHz):δ=2,65-2,82 (m, 2H,), 3.41 (t, 2H), 6.7 (broad s, 1H), 7.25 (broad s, 1H).
MS (DCI,NH₃): 201 (0,2 %, M⁺), 119(50%), 93 (58%), 60 (100%).

### Beispiel 3: Herstellung von 3,4,4-Trifluoro-3-butenyldithiocarbamat (Ammoniumdithiocarbamat-Verfahren)

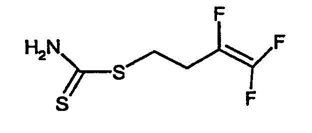

2,8 g (25 mmol) Ammoniumdithiocarbamat werden in 40 ml Ethanol tropfenweise mit 5,32 g (26,3 mmol; Reinheit 93,2%) 4-Brom-1,1,2-trifluor-1-buten versetzt, 3 h bei 40°C gerührt, wobei man nach 1 h nochmals 2,8 g Ammoniumdithiocarbamat hinzugibt, und alles 8 h bei Raumtemperatur stehen lässt. Man saugt ab, dampft das Filtrat im Vakuum ein, nimmt den Rückstand in Dichlormethan auf und wäscht einmal mit Wasser und trocknet die organische Phase mit Natriumsulfat. Nach Eindampfen im Vakuum erhält man 4,3 g (63 % der Theorie; Gehalt nach GC/MS: 74 Flächen-%) eines halbkristallinen Rückstands.

### Beispiel 4: Herstellung von 2-[(3,4,4-Trifluoro-3-butenyl)sulfanyl]-1,3-thiazol (Verfahren (c) mit Chloracetaldehyd)

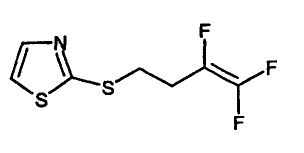

15,1 g (64,8 mmol; Reinheit 86,3 %) 3,4,4-Trifluoro-3-butenyldithiocarbamat werden in 100 ml Dioxan mit 0,2 ml konz. Salzsäure und 12,4 g (71,2 mmol) 45%iger wässriger Chloracetaldehydlösung versetzt. Man kocht 4 h unter Argon, wobei nach 2 h nochmals 1 ml Chloracetaldehydlösung hinzugegeben werden. Anschließend wird im Vakuum eingedampft, der Rückstand in Dichlormethan aufgenommen, mit Wasser gewaschen und die organische Phase nach Trocknung mit MgSO₄ im Vakuum eingedampft. Man erhält 16 g (94,4% der Theorie; Gehalt nach GC/MS: 86,1 Flächen-%) eines Öls, das bei 0,4 T/86°C destilliert werden kann.

### Beispiel 5: Herstellung von 2-[(3,4,4-Trifluoro-3-butenyl)sulfanyl]-1,3-thiazol (Verfahren (c) mit Chloracetaldehyddiethylacetal)

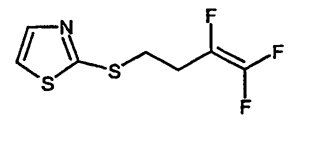

3 g (14,9 mmol) 3,4,4-Trifluoro-3-butenyldithiocarbamat werden in 15 ml Eisessig mit 2,3 g (15,1 mmol) Chloracetaldehyddiethylacetal und 40 mg p-Toluolsulfonsäuremonohydrat versetzt. Man rührt 1,5 h bei 95°C und dampft nach Abkühlung auf Raumtemperatur anschließend im Vakuum ein. Der Eindampfrückstand wird in Dichlormethan aufgenommen, mit 1 N Natronlauge gewaschen, die organische Phase abgetrennt, mit Na₂SO₄ getrocknet und im Vakuum eingedampft. Man erhält 2,8 g (81,5 % der Theorie; Gehalt nach GC/MS: 97,7 Flächen-%) eines Öls.

## Patentansprüche

1. Verfahren zum Herstellen einer Verbindung der Formel (I) 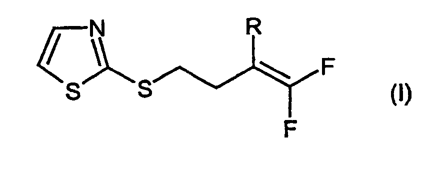 worin
R für H oder F steht, und
**dadurch gekennzeichnet, dass** man
(a) eine Verbindung der Formel (II) 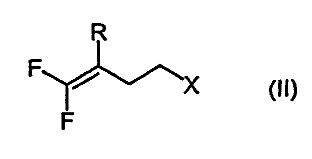 worin
R für H oder F steht, und
X für Brom, Chlor, Mesylat oder Tosylat steht,
mit einer Verbindung der Formel (III)
M⁺ SCN⁻ (III)
worin
M⁺ für Wasserstoff, für ein Ammoniumion, ein Tetraalkylammoniumion oder ein (Erd)Alkalimetallion steht,
gegebenenfalls in Gegenwart eines Reaktionshilfsmittels und gegebenenfalls in Gegenwart eines Verdünnungsmittels zu Verbindungen der Formel (IV) 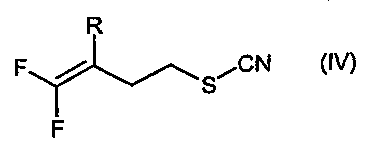 worin
R die vorstehend angegebene Bedeutung hat,
umsetzt,
(b) diese anschließend durch Zuführung von Schwefelwasserstoff oder dessen Salzen,
gegebenenfalls in Gegenwart eines Reaktionshilfsmittels und gegebenenfalls in Gegenwart eines Verdünnungsmittels,
in eine Verbindung der Formel (V) 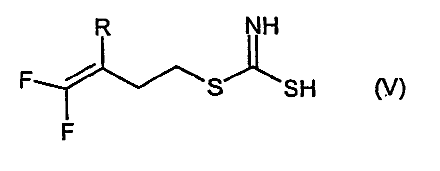 worin
R die vorstehend angegebene Bedeutung hat,
überführt, und
(c) diese mit Acetaldehyd, Chloracetaldehyd (ClCH₂CHO) oder dessen Acetalen oder cyclischen Acetalen gegebenenfalls in Gegenwart eines sauren Reaktionshilfsmittels und gegebenenfalls in Gegenwart eines Verdünnungsmittels, umsetzt.

2. Verfahren zum Herstellen einer Verbindung der Formel (IV) 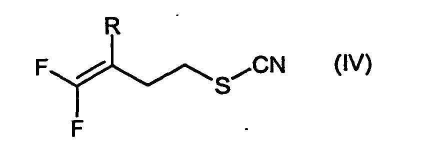 worin
R die vorstehend angegebene Bedeutung hat,
**dadurch gekennzeichnet, dass** man Verbindungen der Formel (II) 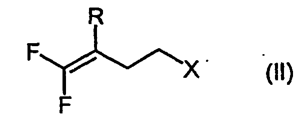 worin R und X die in Anspruch 1 angegebene Bedeutung haben,
mit einem Rhodanidsalz der Formel (III)
M⁺ SCN⁻ (III)
worin
M⁺ die in Anspruch 1 angegebene Bedeutung hat,
gegebenenfalls in Gegenwart eines Reaktionshilfsmittels und gegebenenfalls in Gegenwart eines Verdünnungsmittels umsetzt.

3. Verfahren zum Herstellen von Verbindungen der Formel (V) 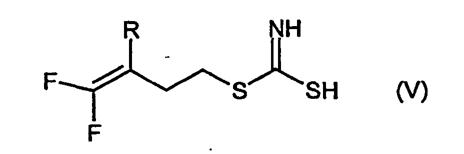 worin
R die in Anspruch 1 angegebene Bedeutung hat,
**dadurch gekennzeichnet, dass** man eine Verbindung der Formel (IV) 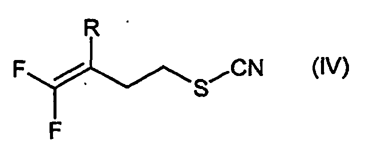 worin
R die in Anspruch 1 angegebene Bedeutung hat,
mit Schwefelwasserstoff oder dessen Salzen gegebenenfalls in Gegenwart eines Reaktionshilfsmittels und gegebenenfalls in Gegenwart eines Verdünnungsmittels umsetzt.

4. Verfahren zum Herstellen einer Verbindung der Formel (I) gemäß Anspruch 1, **dadurch gekennzeichnet, dass** man eine Verbindung der Formel (V) 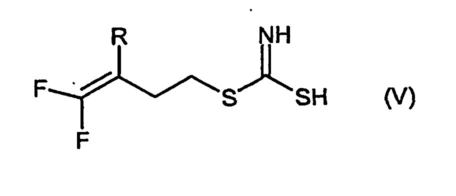 worin
R die in Anspruch 1 angegebene Bedeutung hat,
mit Acetaldehyd, Chloracetaldehyd (ClCH₂CHO) oder dessen Acetale gegebenenfalls in Gegenwart eines Verdünnungsmittels und gegebenenfalls in Gegenwart eines sauren Reaktionshilfsmittels umsetzt.

5. Verfahren gemäß Anspruch 1 oder 2, **dadurch gekennzeichnet, dass** man eine Verbindung der Formel (II) mit HSCN bei Gegenwart einer Base umsetzt.

6. Verfahren gemäß Anspruch 1 oder 2, **dadurch gekennzeichnet, dass** man eine Verbindung der Formel (II) mit NH₄SCN umsetzt.

7. Verfahren gemäß Anspruch 6, **dadurch gekennzeichnet, dass** als Verdünnungsmittel bei der Umsetzung ein Alkohol verwendet wird.

8. Verfahren gemäß Anspruch 1 oder 3, **dadurch gekennzeichnet, dass** man eine Verbindung der Formel (IV) mit H₂S umsetzt.

9. Verfahren gemäß einem der Ansprüche 1, 3 oder 8, **dadurch gekennzeichnet, dass** die Umsetzung in Gegenwart einer Base durchgeführt wird.

10. Verfahren gemäß Anspruch 1 oder 4, **dadurch gekennzeichnet, dass** man eine Verbindung der Formel (V) mit Chloracetaldehyddialkylacetal umsetzt.

11. Verfahren gemäß einem der Ansprüche 1, 4 oder 10, **dadurch gekennzeichnet, dass** die Umsetzung in Gegenwart einer Säure erfolgt.

12. Verfahren gemäß Anspruch 1 oder 4, **dadurch gekennzeichnet, dass** man eine Verbindung der Formel (V) mit Chloracetaldehyd oder dessen Acetale in Gegenwart von 0,1 bis 10 mol-% p-Toluolsulfonsäure oder Methansulfonsäure umsetzt.

13. Verfahren gemäß Anspruch 1 oder 4, **dadurch gekennzeichnet, dass** man eine Verbindung der Formel (V) mit Acetaldehyd umsetzt.

14. Verfahren gemäß einem der Ansprüche 1 bis 13, **dadurch gekennzeichnet, dass** R für Fluor steht.

15. Verbindungen der Formel (IV) 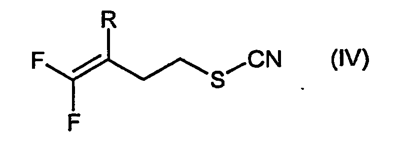 worin
R die in Anspruch 1 angegebene Bedeutung hat.

16. Verbindungen der Formel (V) 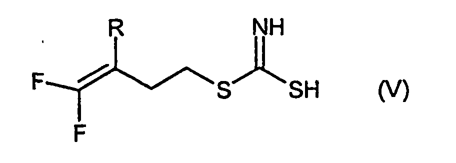 und deren Salze
worin
R für Wasserstoff steht.

## Claims

1. Process for preparing a compound of the formula (I) 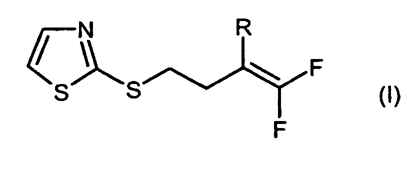 where
R isHorF,
**characterized in that**
(a) a compound of the formula (II) 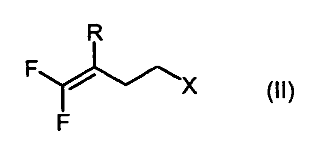 where
R is H or F and
X is bromine, chlorine, mesylate or tosylate,
is reacted with a compound of the formula (III)
M⁺ SCN- (III)
where
M⁺ is hydrogen, an ammonium ion, a tetraalkylammonium ion or an alkali metal or alkaline earth metal ion
optionally in the presence of a reaction auxiliary and optionally in the presence of a diluent to give compounds of the formula (IV) 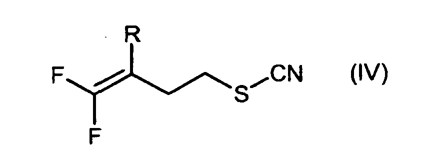 where
R is as defined above,
(b) the latter is then converted by adding hydrogen sulphide or salts thereof,
optionally in the presence of a reaction auxiliary and optionally in the presence of a diluent,
to a compound of the formula (V) 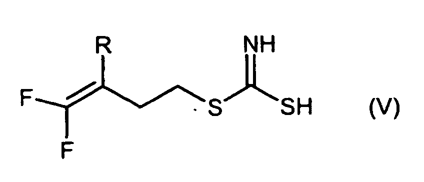 where
R is as defined above,
and
(c) the latter is reacted with acetaldehyde, chloroacetaldehyde (ClCH₂CHO) or the acetals or cyclic acetals thereof, optionally in the presence of an acidic reaction auxiliary and optionally in the presence of a diluent.

2. Process for preparing a compound of the formula (IV) 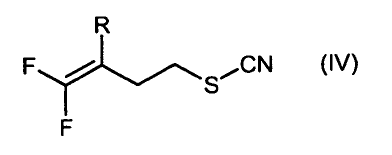 where
R is as defined above,
**characterized in that** compounds of the formula (II) 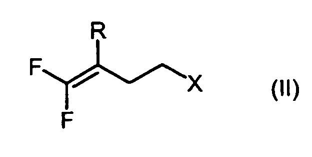 where R and X are as defined in Claim 1
are reacted with a thiocyanate salt of the formula (III)
where
M⁺ is as defined in Claim 1,
optionally in the presence of a reaction auxiliary and optionally in the presence of a diluent.

3. Process for preparing compounds of the formula (V) 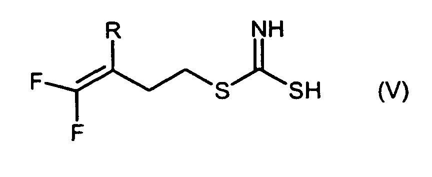 where
R is as defined in Claim 1,
**characterized in that** a compound of the formula (IV) 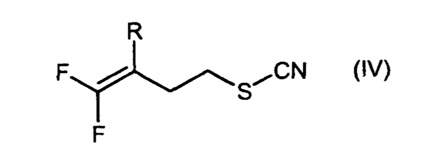 where
R is as defined in Claim 1
is reacted with hydrogen sulphide or salts thereof, optionally in the presence of a reaction auxiliary and optionally in the presence of a diluent.

4. Process for preparing a compound of the formula (I) according to Claim 1, **characterized in that** a compound of the formula (V) 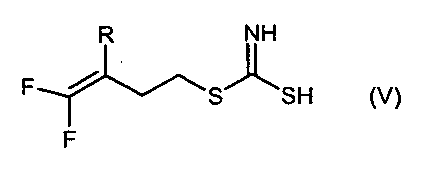 where
R is as defined in Claim 1
is reacted with acetaldehyde, chloroacetaldehyde (ClCH₂CHO) or acetals thereof, optionally in the presence of a diluent and optionally in the presence of an acidic reaction auxiliary.

5. Process according to Claim 1 or 2, **characterized in that** a compound of the formula (II) is reacted with HSCN in the presence of a base.

6. Process according to Claim 1 or 2, **characterized in that** a compound of the formula (II) is reacted with NH₄SCN.

7. Process according to Claim 6, **characterized in that** the diluent used in the reaction is an alcohol.

8. Process according to Claim 1 or 3, **characterized in that** a compound of the formula (IV) is reacted with H₂S.

9. Process according to any of Claims 1, 3 and 8, **characterized in that** the reaction is carried out in the presence of a base.

10. Process according to Claim 1 or 4, **characterized in that** a compound of the formula (V) is reacted with chloroacetaldehyde dialkyl acetal.

11. Process according to any of Claims 1, 4 and 10, which is carried out in the presence of an acid.

12. Process according to Claim 1 or 4, **characterized in that** a compound of the formula (V) is reacted with chloroacetaldehyde or acetals thereof in the presence of from 0.1 to 10 mol% of p-toluenesulphonic acid or methanesulphonic acid.

13. Process according to Claim 1 or 4, **characterized in that** a compound of the formula (V) is reacted with acetaldehyde.

14. Process according to any of Claims 1 to 13, **characterized in that** R is fluorine.

15. Compounds of the formula (IV) 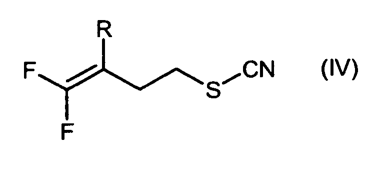 where
R is as defined in Claim 1.

16. Compounds of the formula (V) 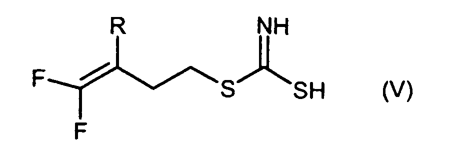 and salts thereof
where
R is hydrogen.

## Revendications

1. Procédé de production d'un composé de formule (I) 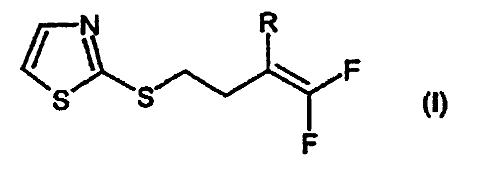 dans laquelle
R représente H ou F,
**caractérisé en ce que**
(a) on fait réagir un composé de formule (II) 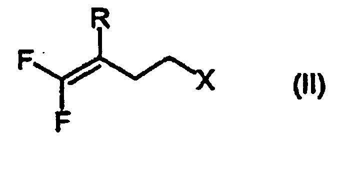 dans laquelle
R représente H ou F et
X représente le brome, le chlore, un reste méthylate ou tosylate,
avec un composé de formule (III)
**M**^{**+**}**SCN**^{**-**} **(III)**
dans laquelle
M⁺ représente l'hydrogène, un ion ammonium, un ion tétra-alkylammonium ou un ion de métal alcalin ou alcalino-terreux,
éventuellement en présence d'un auxiliaire de réaction et, le cas échéant, en présence d'un diluant, pour obtenir des composés de formule (IV) 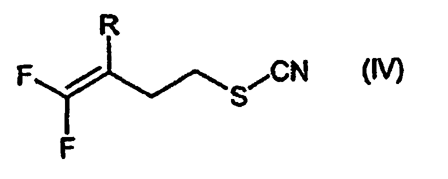 dans laquelle
R a la définition indiquée ci-dessus,
(b) on transforme ensuite ces composés par l'apport de sulfure d'hydrogène ou de ses sels,
éventuellement en présence d'un auxiliaire de réaction et, le cas échéant, en présence d'un diluant, en un composé de formule (V) 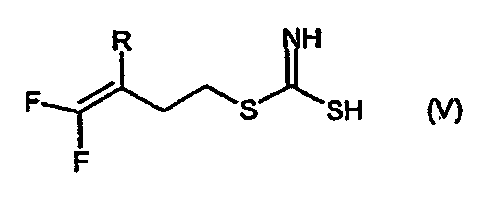 dans laquelle
R a la définition indiquée ci-dessus,
et
(c) on fait réagir ce composé avec l'acétaldéhyde, le chloracétaldéhyde (ClCH₂CHO) ou ses acétals ou acétals cycliques, éventuellement en présence d'un auxiliaire acide de réaction et, le cas échéant, en présence d'un diluant.

2. Procédé de production d'un composé de formule (IV) 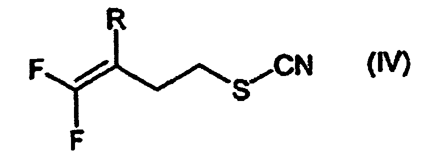 dans laquelle
R a la définition indiquée ci-dessus,
**caractérisé en ce qu'**on fait réagir des composés de formule (II) 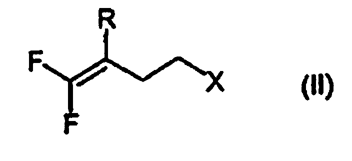 dans laquelle R et X ont à la définition indiquée dans la revendication 1,
avec un sulfocyanure de formule (III)
**M**^{**+**}**SCN**^{**-**} **(III)**
dans laquelle
M⁺ a la définition indiquée dans la revendication 1,
éventuellement en présence d'un auxiliaire de réaction et, le cas échéant, en présence d'un diluant.

3. Procédé de production de composés de formule (V) 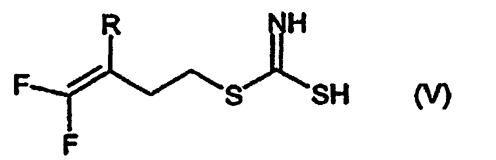 dans laquelle
R a la définition indiquée dans la revendication 1,
**caractérisé en ce qu'**on fait réagir un composé de formule (IV) 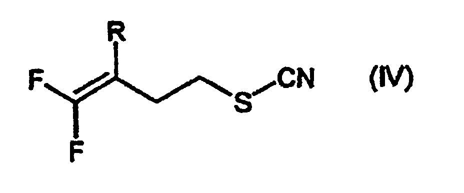 dans laquelle
R a la définition indiquée dans la revendication 1,
avec le sulfure d'hydrogène ou ses sels, éventuellement en présence d'un auxiliaire de réaction et, le cas échéant, en présence d'un diluant.

4. Procédé de production d'un composé de formule (I) suivant la revendication 1, **caractérisé en ce qu'**on fait réagir un composé de formule (V) 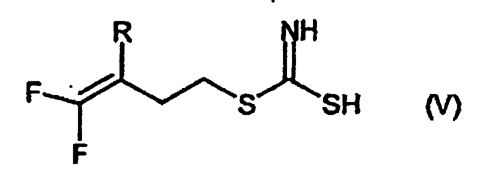 dans laquelle
R a la définition indiquée dans la revendication 1,
avec l'acétaldéhyde, le chloracétaldéhyde (ClCH₂CHO) ou ses acétals, éventuellement en présence d'un diluant et, le cas échéant, en présence d'un auxiliaire acide de réaction.

5. Procédé suivant la revendication 1 ou 2, **caractérisé en ce qu'**on fait réagir un composé de formule (II) avec HSCN en présence d'une base.

6. Procédé suivant la revendication 1 ou 2, **caractérisé en ce qu'**on fait réagir un composé de formule (II) avec NH₄SCN.

7. Procédé suivant la revendication 6, **caractérisé en ce qu'**on utilise un alcool comme diluant dans la réaction.

8. Procédé suivant la revendication 1 ou 3, **caractérisé en ce qu'**on fait réagir un composé de formule (IV) avec H₂S.

9. Procédé suivant l'une des revendications 1, 3 ou 8, **caractérisé en ce qu'**on conduit la réaction en présence d'une base.

10. Procédé suivant la revendication 1 ou 4, **caractérisé en ce qu'**on fait réagir un composé de formule (V) avec un dialkylacétal de chloracétaldéhyde.

11. Procédé suivant l'une des revendications 1, 4 ou 10, **caractérisé en ce qu'**on conduit la réaction en présence d'un acide.

12. Procédé suivant la revendication 1 ou 4, **caractérisé en ce qu'**on fait réagir un composé de formule (V) avec le chloracétaldéhyde ou ses acétals en présence de 0,1 à 10 mol-% d'acide p-toluènesulfonique ou d'acide méthanesulfonique.

13. Procédé suivant la revendication 1 ou 4, **caractérisé en ce qu'**on fait réagir un composé de formule (V) avec l'acétaldéhyde.

14. Procédé suivant l'une des revendications 1 à 13, **caractérisé en ce que** R représente le fluor.

15. Composés de formule (IV) 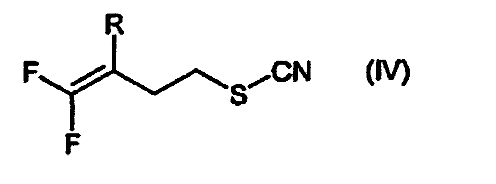 dans laquelle
R a la définition indiquée dans la revendication 1.

16. Composés de formule (V) 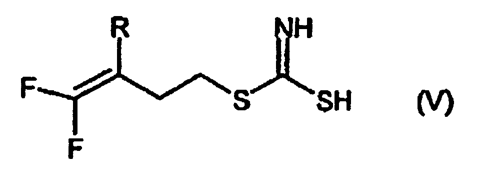 et ses sels,
formule dans laquelle
R représente l'hydrogène.
